# EUROPEAN PATENT SPECIFICATION

(11) **EP 0 602 173 B1**
(45) Date of publication and mention of the grant of the patent: **26.05.1999**
(21) Application number: 92919794.5
(22) Date of filing: 04.09.1992
(51) Int. Cl.: C07K 1/30, C07K 1/22, C07K 14/75, A61K 38/43, A61K 38/36, A61K 38/38, A61K 38/45, A61K 38/55, A61K 38/57, A61K 38/58

(54) **TOPICAL FIBRINOGEN COMPLEX**
TOPISCHER FIBRINOGENKOMPLEX
COMPOSITION A BASE DE FIBRINOGENE POUR APPLICATION TOPIQUE

(30) Priority: 05.09.1991 US 755156
(43) Date of publication of application: 22.06.1994
(73) Proprietor: BAXTER INTERNATIONAL INC., Deerfield, IL 60015 (US)
(72) Inventor: TSE, Daphne, C., Duarte, CA 91010 (US); MANKARIOUS, Samia, S., Costa Mesa, CA 92626 (US); LIU, Shu, Len, Cerritos, CA 90701 (US); THOMAS, William, R., Laguna Nigel, CA 92677 (US); ALPERN, Melaine, Long beach, CA 90808 (US); ENOMOTO, Stanley, T., Van Nuys, CA 91406 (US); GARANCHON, Cataline, M., Northridge, CA 91325 (US)
(74) Representative: Thomas, Philip John Duval
(86) International application number: US9207493
(87) International publication number: WO9305067

(56) References cited:
- EP-A- 0 408 029
- WO-A-86/01814
- JP-A-49 019 011
- US-A- 4 175 182
- US-A- 4 362 567
- US-A- 4 427 650
- US-A- 4 442 655
- US-A- 4 627 879
- US-A- 5 094 960
- US-A- 5 138 034
- REMINGTON's PHARMACEUTICAL SCIENCES, 15th ed., 1975; Chapter 67, p. 1252
- THROMBOSIS RESEARCH, vol. 1, 1972; M. MATSUDA et al., pp. 619-630

## Description

This application is a continuation-in-part of U.S. Serial No. 755,156, filed September 5, 1991.

### BACKGROUND OF THE INVENTION

### Field of The Invention

The present invention relates to a fibrinogen composition and its method of preparation, wherein the composition can be used for wound closure in conjunction with thrombin and calcium.

### Related Art

The attempt to use fibrinogen to achieve topical hemostasis was investigated as far back as the early 20th Century when fibrinogen patches for hemostasis were used in cerebral surgery. Later, mixtures of plasma and thrombin were used for skin grafting and intracavity injections in the therapy of tuberculosis. However, these early attempts had two major drawbacks: Since the source of fibrinogen was plasma, the concentration of fibrinogen was low which resulted in a fibrin film of insufficient strength; and it was not possible to inhibit the normal physiologic process of fibrinolysis such that the fibrin film was degraded relatively quickly.

Prior attempts to develop an effective fibrin sealant have also been hampered by the fact that most of these preparations contain high levels of plasminogen which required these compositions to additionally contain an anti-fibrinolytic agent in order to prevent premature degradation of the fibrin seal. Because anti-fibrinolytic agents are typically derived from a non-human source the possibility of a patient having an adverse reaction to such foreign proteins is significant, especially upon multiple exposure to these agents.

Although Rose, *et al.* (U.S. 4,627,879) report the production of a fibrin adhesive which does not necessarily require the presence of an anti-fibrinolytic additive, the composition disclosed in this reference does not deal with another major drawback of these prior fibrin sealing compositions, namely, the possible presence of infectious agents, such as Hepatitis B or HIV, in the plasma. As a consequence, the Rose reference requires that the compositions described therein be derived from a single donor, in order to avoid the transmission of infectious agents which might be associated with pooled plasma.

Thus, there is considerable need for a fibrin sealant which can be derived from pooled plasma and which is free of anti-fibrinolytic compounds, animal proteins, and infectious agents such as viruses. The present invention addresses these needs by providing such compositions.

### SUMMARY OF THE INVENTION

The present invention is based upon the discovery that pooled plasma, even when substantially depleted of Factor VIII, can be processed to produce a fibrinogen preparation which reacts with thrombin and calcium, to produce a fibrin sealant that can be used to promote hemostasis.

In detail, the invention provides a fibrinogen composition which, in addition to being essentially free of Factor VIII and plasminogen, does not require the use of an anti-fibrinolytic agent and has been treated to eliminate the presence of infectious agents such as lipid enveloped viruses. A further advantage of the composition is that essentially all of the proteins present in the composition are human, i.e., encoded by a human DNA sequence.

The composition of the invention, through its transient *in vivo* presence, provides a matrix which persists for a period of time sufficient to achieve a medical effect, essentially lacks host toxicity upon degradation, and provides mechanical strength to promote hemostasis.

### BRIEF DESCRIPTION OF THE DRAWINGS

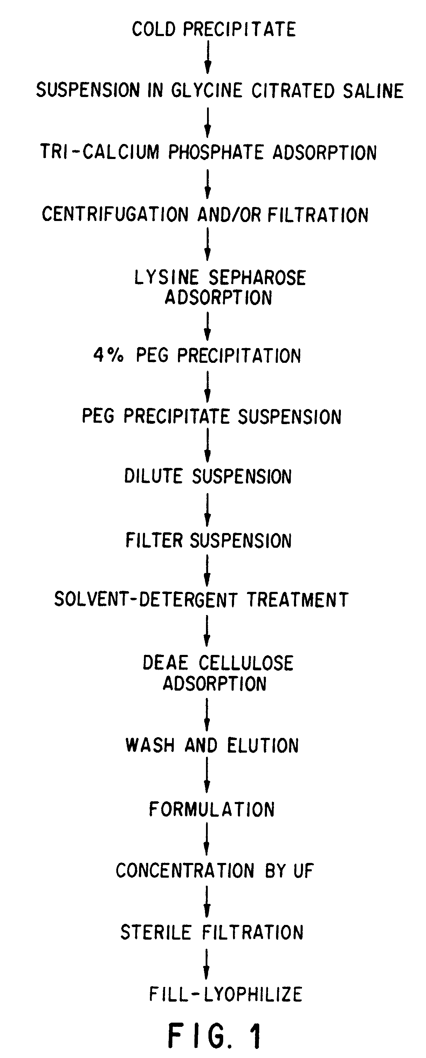

FIGURE 1 Schematic representation for preparation of Topical Fibrinogen Complex.

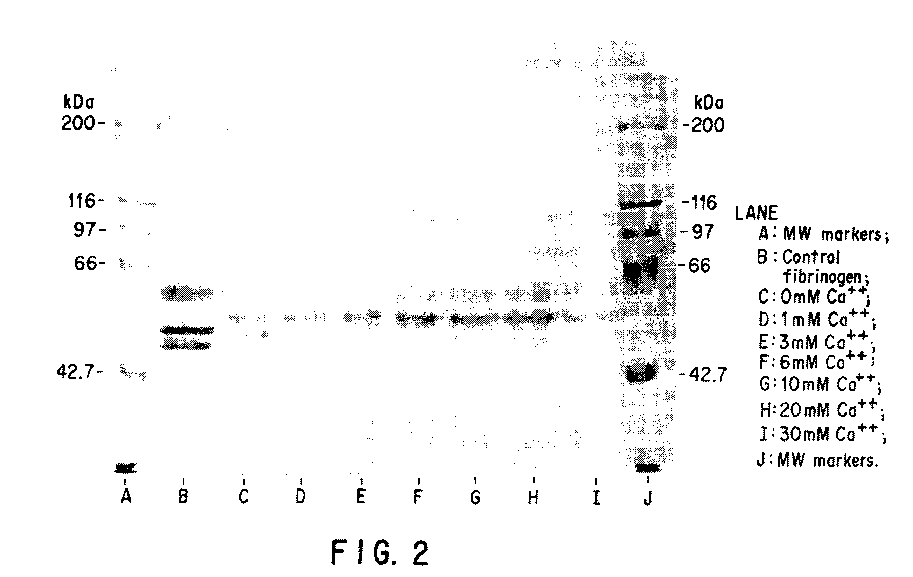

FIGURE 2 Effect of calcium ion concentration on fibrin polymer formation. Fibrinogen (90mg/ml) and thrombin (500U/ml) were mixed and allowed to incubate for 10 minutes. Lane A: MW markers; Lane B: Control fibrinogen; Lane C: 0mM Ca⁺⁺; Lane D: 1mM Ca⁺⁺; Lane E: 3mM Ca⁺⁺; Lane F: 6mM Ca⁺⁺; Lane G: 10mM Ca⁺⁺; Lane H: 20mM Ca⁺⁺; Lane : 30mM Ca⁺⁺; Lane J: MW markers.

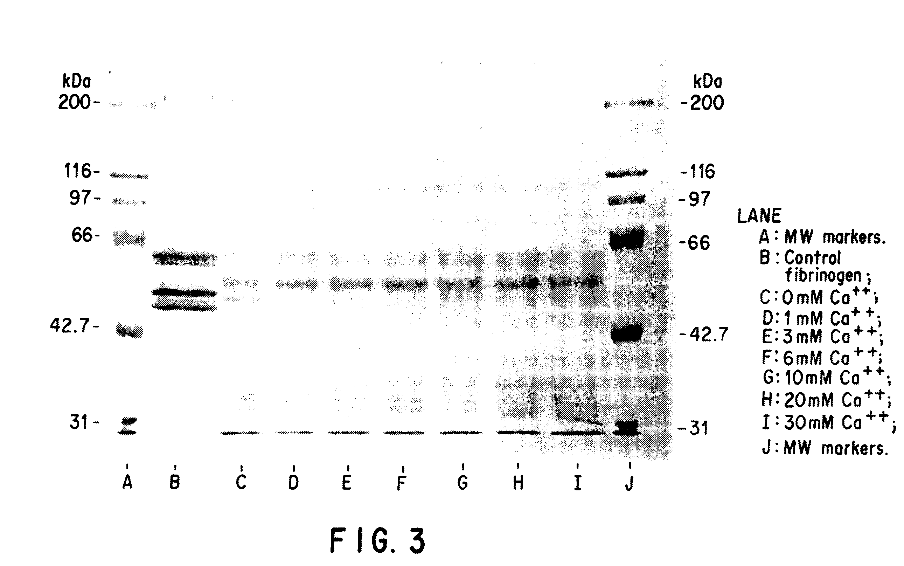

FIGURE 3 Effect of calcium ion concentration on fibrin polymer formation. Fibrinogen (130 mg/ml) and thrombin (500 U/ml) were mixed and allowed to incubate for 10 minutes. Lane A: MW markers; Lane B: Control fibrinogen; Lane C: 0 mM Ca⁺⁺; Lane D: 1mM Ca⁺⁺; Lane E: 3mM Ca⁺⁺; Lane F: 6mM Ca⁺⁺; Lane G: 10mM Ca⁺⁺; Lane H: 20mM Ca⁺⁺; Lane I: 30mM Ca⁺⁺; Lane J: MW markers.

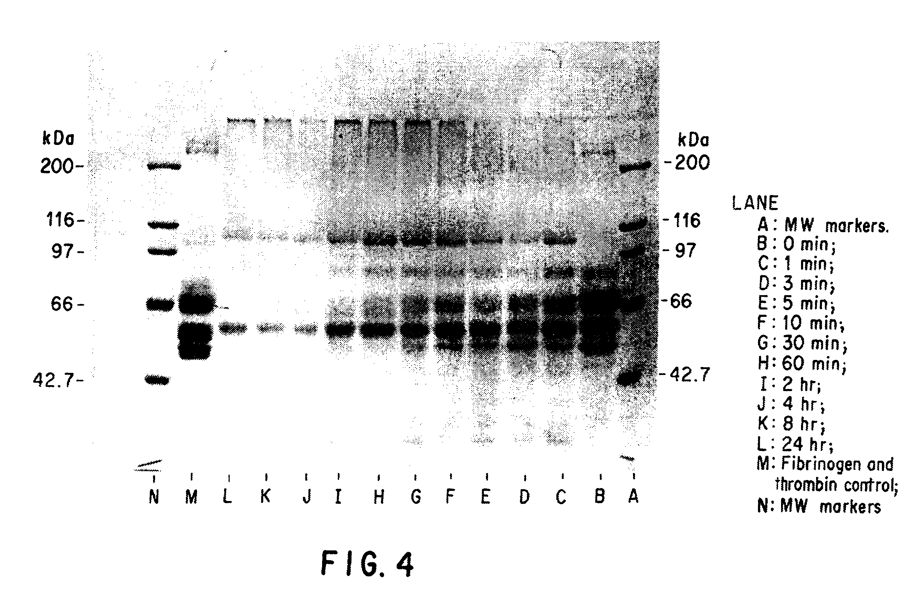

FIGURE 4 Rate of fibrin polymerization. Fibrinogen (130 mg/ml) and thrombin (500 U/ml) were mixed in the presence of calcium ion (40mM CaCl₂). Lane A: MW markers; Lane B: 0 min; Lane C: 1 min; Lane D: 3 min; Lane E: 5 min; Lane F: 10 min; Lane G: 30 min; Lane H: 60 min; Lane I: 2 hr; Lane J: 4 hr; Lane K: 8 hr; Lane L: 24 hr; Lane M: fibrinogen and thrombin control; Lane N: MW markers.

### DETAILED DESCRIPTION OF THE PREFERRED EMBODIMENT

The inventors have devised compositions for producing a fibrin sealant which represent a significant improvement over the prior art compositions intended to accomplish this effect. These compositions, termed Topical Fibrinogen Complex (TFC), are advantageous because they are able in the absence of non-human proteins and possible viral pathogens to achieve *in situ* longevity in order to induce hemostasis.

The compositions of the invention can be produced by starting with any blood-derived fraction which has not been significantly depleted of fibrinogen. Preferred blood fractions for producing the compositions of the invention are plasma, cryoprecipitate, and Factor VIII-depleted cold-precipitate. Generally, the process involves the formation of a cryoprecipitate which is high in Factor VIII (F VIII) and fibrinogen. This step is followed by a cold-precipitation which, in turn, contains a high concentration of fibrinogen and very low levels of F VIII. The addition of a calcium ion source during the cold-precipitation step enhances the precipitation of fibrinogen, as well as fibronectin, thereby increasing the concentration of these substances in the cold-precipitate. The addition of polyethylene glycol (PEG) can also be used to concentrate the fibrinogen in the composition.

More specifically, the method of the present invention typically starts with containers of frozen plasma. As described in Example 1, the cryoprecipitate is collected from the frozen plasma following thawing in a controlled environment.

Prior to formation of the cold-precipitate, the blood-fraction is treated to produce a cryoprecipitate. This cryoprecipitate can be produced by freezing the blood fraction (for example, plasma) which is thereafter warmed to a temperature not exceeding about +6°C. The cryoprecipitate is dissolved in distilled water at about 20°C-35°C. Calcium chloride is added at a concentrate from about 1 µM to about 1000 µM. Preferably, calcium chloride is added at this step at a concentration of about 40 µM and the pH adjusted to 6.8 ± 0.3 to enhance the precipitation of fibrinogen.

The dissolved cryoprecipitate is then cooled to 10°C ± 2°C with mixing, whereupon a precipitate, known as the cold-precipitate. forms. The precipitate is removed from solution by centrifugation, for example, 5600 g to 7200 g. The precipitate may be stored at -60°C or lower if desired. In the preferred embodiment, the initial fractionation to obtain the topical fibrinogen complex is accomplished by first performing a cryoprecipitation step followed by the cold-precipitation step.

The resulting precipitate, rich in fibrinogen, is dissolved in buffer with mixing and the pH is adjusted to 7.2 ± 0.1. Preferably the cold precipitate is resuspended in the presence of a protease inhibitor, such as PPACK (D-Phe-L-pro-L-arg-chloromethylketone), heparin cofactor II, or hirudin to inhibit thrombin which may be present. Most preferred as thrombin inhibitor is PPACK at a concentration from about 0.75 µM to about 1.75 µM. Thrombin inhibitor is removed in the PEG precipitation and DEAE column steps of the procedure. Those of skill in the art will know of other protease inhibitors of thrombin and their effective concentrations.

The cold precipitate suspension is transferred to a buffer solution containing a salt such as tri-calcium phosphate. The calcium phosphate removes prothrombin from the fibrinogen rich solution. This minimizes the likelihood of prothrombin conversion to thrombin which, if such a reaction were to occur, could lead to the conversion of fibrinogen to fibrin. The calcium phosphate, in turn, is removed from the process by centrifugation and/or filtration. Additional techniques for removal of prothrombin are described by Murano *(Prothrombin and Other Vitamin K Proteins,* Vol. II, Seegers and Walz, eds., CRC Press, Inc. Boca Raton, Fl., 1986); Heystek, *et al. (Vox Sang.,* 25:113, 1973); Banowcliffe, *et al. (Vox Sang.,* 25:426, 1973); Chandra, *et al. (Vox Sang.,* 41:257, 1981); and Chanas, *et al.* (US. 4,465,623), incorporated herein by reference.

In a preferred embodiment, the dissolved cold-precipitate is warmed to about 23-27°C and contacted with lysine-Sepharose 48. The fibrinogen rich fraction, which essentially does not bind to the lysine-Sepharose 4B, is separated from the affinity absorbent. The dissolved prothrombin-free cold-precipitate is contacted with lysine bound to a solid matrix, such as Sepharose, to allow adsorption of residual plasminogen in the cold-precipitate. The removal of plasminogen is important since it is a profibrinolytic zymogen which can break down fibrinogen and fibris molecules. The material which is not bound to the lysine matrix is then concentrated, preferably by the addition of polyethylene glycol (PEG). Polyethylene glycol (3000-8000 MW) is then added to a final concentration from about 3% to about 7%, preferably at about 4% (W/W). The PEG precipitate is dissolved and the pH adjusted to 8.6 ± 0.1 before clarification by filtration. When PEG is used for the concentration step, the weight-range of the PEG should be that which is non-toxic in humans. In an alternate embodiment of the invention, it is also possible to perform the PEG precipitation step prior to adsorption with the lysine matrix.

After filtration, the composition is treated with a virally inactivating effective amount of an agent, such as a detergent, which, typically, acts by disrupting the lipid envelope of such viruses as Hepatitis B, HIV, and HTLV. The term "virally inactivating effective amount" means that the concentration of detergent added to the composition is sufficient to inactivate essentially all virions which might be present. Of course, the concentration of detergent should not significantly inhibit the ability of the composition to function adequately in formation of the fibrin glue. Detergents which are useful can be selected from such recognized groups as anionic, cationic, and non-ionic detergents. Examples include sulfated alcohols and sodium acid salts, such 1973); Banowcliffe, *et al. (Vox Sang.,* 25:426, 1973); Chandra, *et al. (Vox Sang.,* 41:257, 1981); and Chanas, *et al.* (US. 4,465,623).

In a preferred embodiment, the dissolved cold-precipitate is warmed to about 23-27°C and contacted with lysine-Sepharose 4B. The fibrinogen rich fraction, which essentially does not bind to the lysine-Sepharose 4B, is separated from the affinity absorbent. The dissolved prothrombin-free cold-precipitate is contacted with lysine bound to a solid matrix, such as Sepharose, to allow adsorption of residual plasminogen in the cold-precipitate. The removal of plasminogen is important since it is a profibrinolytic zymogen which can break down fibrinogen and fibris molecules. The material which is not bound to the lysine matrix is then concentrated, preferably by the addition of polyethylene glycol (PEG). Polyethylene glycol (3000-8000 MW) is then added to a final concentration from about 3% to about 7%, preferably at about 4% (W/W). The PEG precipitate is dissolved and the pH adjusted to 8.6 ± 0.1 before clarification by filtration. When PEG is used for the concentration step, the weight-range of the PEG should be that which is non-toxic in humans. In an alternate embodiment of the invention, it is also possible to perform the PEG precipitation step prior to adsorption with the lysine matrix.

After filtration, the composition is treated with a virally inactivating effective amount of an agent, such as a detergent, which, typically, acts by disrupting the lipid envelope of such viruses as Hepatitis B, HIV, and HTLV. The term "virally inactivating effective amount" means that the concentration of detergent added to the composition is sufficient to inactivate essentially all virions which might be present. Of course, the concentration of detergent should not significantly inhibit the ability of the composition to function adequately in formation of the fibrin glue. Detergents which are useful can be selected from such recognized groups as anionic, cationic, and non-ionic detergents. Examples include sulfated alcohols and sodium acid salts, such as sulfated oxyethylated alkylphenol (Triton W-30 and Triton X-100), sodium dodecylbenzensulfonate (Nacconol NR), sodium 2-sulfoethyl oleate (Igepon A), sodium cholate, sodium deoxycholate, sodium dodecylsulfonate, dodecyldimethylbenzylammonium chloride (Triton K-60), oxyethylated amines (Ethomeen), N-dodecylaminoethanesulfonic acid, ethylene oxide-propylene oxide condensates (Pluronic copolymers), polyoxyethylated derivatives of esters (Tween 80 and Polysorbate 80), polyoxyethylene fatty alcohol ethers (Brij 35), nonidet P-40 and Lubrox PX. In the preferred embodiment, where the protein concentration is 0.6%, Triton X-100 (final conc. 1.0%), and Polysorbate-80 (final conc. 0.3%) are combined with an organic solvent such as tri (N-butyl) phosphate (TNBP) (final conc. 0.3%) to inactivate viruses. In addition, chaotropic agents may also be utilized to inactivate viruses, providing the use of these agents does not result in significant loss of fibrinogen activity.

The concentration of organic solvent and detergent used in the practice of the preferred embodiments of the invention can vary, depending upon the composition to be treated, and upon the solvent or detergent selected. The alkyl phosphates are used in concentrations from about 0.10 mg/ml of mixture treated to 1.0 mg/ml, preferably between about 0.1 mg/ml to about 10 mg/ml. The amount of detergent or wetting agent utilized is not crucial since its function is to improve the contact between the organic solvent and the virus. For most of the nonionic materials which are useful, the wetting agent can vary from about 0.001% to 10%, preferably from about 0.01% to about 2% of the aqueous mixture, depending upon the amount of fatty material in the treated aqueous mixture. The amounts of solvent and detergent will also vary depending upon each other.

The prior art describes the addition of organic solvents and detergents to concentrated protein solutions in order to disrupt and inactivate lipid-enveloped viruses while preserving the structure and activity of the protein for example, U.S. Patent 4,540,573 reference. Organic solvents containing either Tween-80 or Triton X-100 have been shown to be effective reagents for killing viruses found in concentrated solutions of proteins without adversely affecting the activity of the proteins. However, the use of such solvent-detergent mixtures has traditionally been avoided because the subsequent removal of the detergent mixture from the concentrated protein has been shown to be difficult. As a result, other detergents, such as sodium cholate or sodium deoxycholate, have been more commonly used. These detergents can be removed from the composition by gel exclusion chromatography, for example, using Sephadex G-25. In any event, in selecting a given detergent, or mixture of detergents, it is important to avoid using those detergents which would denature fibrinogen in such manner as to prevent its ability to participate in clot formation.

In the preferred embodiment, DEAE diethylaminoethyl cellulose (DE 52) is the matrix utilized for the removal of the solvent/detergent from the fibrinogen composition. The fibrinogen binds to the DE 52 cellulose and, after thorough washing to remove unbound material and detergent, is eluted with 0.3M NaCI. Other ion exchange materials which can be utilized for removal of the solvent/detergent include virtually any of the commercially available anion exchange matrices, including celluloses and agaroses, such as polysulfated agaroses, specifically included but not limited to QAE (quaternary amine) derivatives, ecteola (epichlorohydrintriethanolamine), TEAE (triethylaminoethyl) derivatives, and AE (aminoethyl) matrices. The specific parameters for binding and eluting from these various ion exchange materials are known to those of skill in the art, or can be readily ascertained, without undue experimentation.

Upon elution from the ion exchange resin, the fibrinogen composition formulation is developed by the addition of such excipients as human serum albumin (HSA) and Polysorbate-80 added to enhance solubility. The addition of HSA, hydroxyethyl starch, dextran, or combinations thereof enhances the stability of the final product. Preferably, the HSA and Polysorbate-80 are added at a concentration of 80mg and 15mg, respectively, per gram of protein in the eluate.

In a preferred embodiment, the fibrinogen composition of the invention is formulated prior to final concentration through ultrafiltration, lyophilization, or other conventional method, in order to enhance the solubility of the final product when reconstituted prior to therapeutic use. Typically, after formulation the bulk is concentrated from about 20% to about 50% of its original eluate volume, then diluted to the pre-concentration eluate volume. The bulk is then concentrated to a final protein concentration of 4±1g%(w/v) before sterile processing and lyophilization.

In the preferred embodiment, concentration is accomplished by ultrafiltration using a membrane with a molecular exclusion large enough to allow NaCI to be removed, but small enough to retain protein molecules. This filtration is most preferably performed using a membrane with a 30,000 MW exclusion. When the TFC composition of the invention is lyophilized, the pre-lyophilization volume is usually greater than the volume to which the lyophilizate is resuspended at time of use. Thus, when the composition is prepared as preferably prescribed above involving ultrafiltration, the reconstituted volume following lyophilization is about 33% of the pre-lyophilization volume. In any event, the NaCI concentration in the reconstituted lyophilizate should preferably be about 0.1M to about 0.2M.

If desired, the compositions of the invention can be modified to include non-proteinaceous as well as proteinaceous drugs. The term "non-proteinaceous drugs" encompasses compounds which are classically referred to as drugs, such as mitomycin C, daunorubicin, and vinblastine as well as antibiotics.

The proteinaceous drugs which can be added to the fibrinogen compositions of the invention include immunomodulators and other biological response modifiers. The term "biological response modifiers" is meant to encompass substances which are involved in modifying a biological response, such as the immune response or tissue growth and repair, in a manner which enhances a particular desired therapeutic effect, for example, the cytolysis of bacterial cells or the growth of epidermal cells. Examples of response modifiers include such compounds as lymphokines. Examples of lymphokines include tumor necrosis factor, the interleukins, lymphotoxin, macrophage activating factors, migration inhibition factor, colony stimulating factors, and the interferons. In addition, peptide or polysaccharide fragments derived from these proteinaceous drugs, or independently produced, can also be incorporated into the fibrinogen compositions of the invention. Those of skill in the art will know, or can readily ascertain, other substances which can act as proteinaceous or non-proteinaceous drugs.

The compositions of the invention can also be modified to incorporate a diagnostic agent, such as a radiopaque agent. The presence of such agents allow the physician to monitor the progression of wound healing occurring internally, such as at the liver, gall bladder, urinary tract, bronchi, lungs, heart, blood vessels, and spinal canal. Such compounds include barium sulfate as well as various organic compounds containing iodine. Examples of these latter compounds include iocetamic acid, iodipamide, iodoxamate meglumine, iopanoic acid, as well as diatrizoate derivatives, such as diatrizoate sodium. Other contrast agents which can be utilized in the compositions of the invention can be readily ascertained by those of skill in the art.

The concentration of drug or diagnostic agent in the composition will vary with the nature of the compound, its physiological role, and desired therapeutic or diagnostic effect. The term "therapeutically effective amount" means that the therapeutic agent is present in a sufficient concentration to minimize toxicity, but display the desired effect. Thus, for example, the concentration of an antibiotic used in providing a cytolytic therapeutic effect will likely be different from the concentration of an immune response modulator where the therapeutic effect is to stimulate the proliferation of immune cells at the site of application of the fibrinogen complex. The term "diagnostically effective amount" denotes that concentration of diagnostic agent which is effective in allowing the fibrin glue to be monitored, while minimizing potential toxicity. In any event, the desired concentration in a particular instance for a particular compound is readily ascertainable by one of skill in the art.

The above disclosure generally describes the present invention. A further understanding can be obtained by reference to the following specific examples which are provided herein for purposes of illustration only, and are not intended to be limiting unless otherwise specified.

### EXAMPLE 1

### PREPARATION OF TOPICAL FIBRINOGEN COMPLEX

Topical fibrinogen complex (TFC) was produced by the initial preparation of a cryoprecipitate of plasma. The cryoprecipitate for such use was prepared by two different techniques depending upon the physical form of the plasma.

In one technique, sealed plastic bottles of frozen plasma were thawed in a controlled environment by contact with a heat exchange medium, such as air or water. The thaw was controlled by programming the temperature and flow of the heat-exchange medium so that the maximum temperature of the plasma did not exceed +6°C. The containers were then opened and the contents pooled into a jacketed stainless steel thawing tank. In the thawing tank, the plasma was gently warmed (while being mixed) to melt the remaining ice. The thawed plasma was then pumped directly to a centrifuge or into a jacketed stainless steel holding tank where it was maintained at 2.5°C ± 3.5°C. The plasma was centrifuged to remove the cryoprecipitate. The cryoprecipitate, so prepared, may be stored at or below -25°C or immediately processed to Antihemophilic Factor (Human). The cryo-poor plasma was collected in a jacketed stainless steel reaction tank.

Alternatively, cryoprecipitate was prepared by placing sealed plastic bags of frozen plasma in a liquid nitrogen bath for several seconds. The bags were removed from the bath and the crisp, cracked bags were stripped from the plasma. The plasma was then placed into a jacketed stainless steel thawing tank. Alteratively, sealed plastic bags of frozen plasma were arranged so as to warm the bags so that the frozen plasma would break away from the plastic. The containers were then opened and the contents pooled into a jacketed stainless steel thawing tank. In the thawing tank the plasma was gently warmed, while being mixed, to melt the remaining ice.

The thawed plasma was pumped directly to a centrifuge or into a stainless steel holding tank where it was maintained at 2.5°C ± 3.5°C. The plasma was centrifuged to remove the cryoprecipitate. The cryoprecipitate, so prepared, may be stored at or below -25°C, or immediately be processed to Antihemophilic Factor (Human). The cryo-poor plasma was collected in a jacketed stainless steel reaction tank.

After the cryoprecipitate was prepared, it was dissolved in distilled water at 20°C to 35°C. This part of the protocol is illustrated schematically in FIGURE 1. Sufficient calcium chloride was added to obtain a minimum calcium concentration of about 40 µM and the pH adjusted to 6.8 ± 0.3. This solution was cooled to 10°C ± 2°C while mixing. The precipitate which forms was removed by centrifugation (5600g-7200g). The precipitate may be stored at or below -60°C or processed directly to Topical Fibrinogen Complex (Human). The precipitate was then suspended in Process Solution I at a ratio of four liters of Process Solution I per kg of precipitate. Process Solution I comprises: (a) 0.5M glycine, 0.5M sodium chloride, and 0.1M sodium citrate (pH adjusted to 7.2 ± 0.1 with NaOH); (b) Protease Inhibitor: 0.75-1.75 µM PPACK (D-phe-L-pro-L-arg-chloromethyl ketone) or equivalent, and 0.6 ± 0.1 U/ml Heparin. The temperature was adjusted to 24-32°C and the suspension stirred for approximately one hour.

After the precipitate was suspended in Process Solution I, the suspension was transferred into a tank containing 200 l of process Solution II at 10-15°C and stirred for at least 30 minutes. Process Solution II comprises: 7 ± 1 mM sodium phosphate monobasic monohydrate, 18 ± 2 mM sodium phosphate dibasic heptahydrate, calcium phosphate tribasic 0.25% (w/v). The suspension was then allowed to settle undisturbed for at least 30 minutes. The suspension may be centrifuged (5600g-7200g) at this step to remove some precipitate. The suspension was then clarified by filtration first through a 0.45µ filter, then through a 0.2µ filter.

The filtrate was warmed to 23-27°C and applied to a Lysine Sepharose 4B column or equivalent. The gel was packed in a chromatography column, prepared with 10 column volumes of 0.1M acetic acid, then 3 column volumes of distilled water, and was then equilibrated with 5 column volumes of Process Solution III (25 mM phosphate buffer: 7 ± 1 mM sodium phosphate monobasic monohydrate, 18 ± 2 mM sodium phosphate dibasic heptahydrate). If desired, the column can be reused upon regeneration with 3 column volumes of 1.0M NaCI, then 3 column volumes of distilled water, then 3 column volumes of 0.1 N acetic acid, and then 3 column volumes of distilled water. For long term storage, the column can be washed with 3 column volumes of 20% ethanol in distilled water and stored. The unbound material was collected in a tank. The column was washed with at least two gel volumes of Process Solution III. The unbound fractions were pooled. Next, the temperature of the bulk containing the pooled unbound fractions was adjusted to 14 ± 4°C. Polyethylene glycol 3350 was added to a final concentration of 4% (w/w). The suspension was mixed for at least 30 minutes and the precipitate removed by centrifugation (8700g) at 10-18°C. The resulting PEG precipitate was dissolved in Process Solution IV (39 mM Iris-phosphate with pH adjusted to 8.6 ± 0.1 with phosphoric acid) approximately 15 l/kg precipitate. The protein concentration of the suspension was adjusted to 0.6 ± 0.2 g% (w/v), then the suspension was clarified by filtration (0.2µ). A mixture of Triton X-100, tri (N-butyl) phosphate (TNBP) and Polysorbate-80 was added to the solution to a final concentration of 1.0% (v/v), 0.3% (v/v), and 0.3% (v/v), respectively. The protein-detergent solution was mixed for 1 hour.

The bulk solution was then applied to a chromatography column containing DE 52 ion exchange cellulose resin or equivalent. The resin was packed in a chromatography column and regenerated with 3 column volumes of 1.0M NaCI, then 3 column volumes of 0.5M HCI, then 3 column volumes of 0.9% saline, and then 3 column volumes of 0.5M NaOH and equilibrated with 3 column volumes of 0.5M Iris-phosphate buffer (pH adjusted to 8.6), and 3 column volumes of Process Solution IV. The column can be reused upon regeneration and can be stored in 0.1M NaOH.

The ion exchange column was then washed with a minimum of 20 column volumes of Process Solution V (0.02M histidine, 0.01M NaCI, pH adjusted to 7.0 ± 0.1 with 6N HCI) until all unbound material and detergent were removed. The bulk was eluted with Process Solution VI (0.02M histidine, 0.3M NaCI, pH adjusted to 7.0 ± 0.1 with 6N HCI).

The bulk was then supplemented with human serum albumin (5% or 25% human albumin released for therapeutic use) to a concentration of 80 mg of albumin per gram of protein. Polysorbate-80 was added to a final concentration of 15 mg per gram of protein.

Finally, the bulk was concentrated to about 25% of the original volume by ultrafiltration using a 30,000 MW cut-off membrane, then diluted to its preconcentration volume with Process Solution VII (0.02M histidine, pH adjusted to 7.0 ± 0.1). The bulk was again concentrated by ultrafiltration to achieve a final protein concentration of 4 ± 1 g% (w/v). The bulk was sterile filtered (0.2µ), aseptically filled into sterile final containers, lyophilized under aseptic conditions, and closed with sterile closures.

### EXAMPLE 2

### TFC in vitro STUDIES

### A. Clotting Evaluation

Studies were done to determine the composition of the Fibrin Sealant (FS) components that will produce fast clotting. Fast clotting was arbitrarily defined as 1-2 seconds. Shorter time periods generally result in clotting within the delivery device and longer times lead to a loose mixture of the components that flows with ill-defined direction.

In performing these experiments, a clean pyrex glass plate was positioned at ~30° from the horizontal axis. A 2" line was drawn on the underside to define the area of FS application.

Human Fibrinogen was tested at 50-130 mg/ml total protein with Bovine Thrombin (Armour Pharmaceutical Co.) at 100-1000 NIH U/ml without Ca⁺⁺. Fibrinogen concentration was considered to equal the value of total proteins. The effect of adding [Ca⁺⁺] to form a Fibrin Sealant (FS; TFC, thrombin and Ca⁺⁺) was studied at 10, 20, 40, and 60 mM.

The Fibrin Sealant was delivered using an experimental dual syringe device (Fenwal) following the 2" line described above, starting at the upper end and moving downward.

Table 1 summarizes the data obtained by testing Fibrinogen at 50-130 mg/ml with Thrombin at 100-1000 NIH U/ml in the absence of Calcium. Each data point is the average of four determinations.

**TABLE 1**

| **AVERAGE CLOTTING TIMES IN SECONDS (±SD)** | | | | |
|---|---|---|---|---|
| | **Thrombin in NIH U/ml** | | | |
| **Total Protein (mg/ml)** | **100** | **250** | **500** | **1000** |
| 50 | 5.3±1.3 | 2.5±0.4 | 2.3±0.4 | 1.2(N=1) |
| 90 | 3.8±0.4 | 2.4±0.4 | 1.9±0.4 | 1.4(N=1) |
| 110 | 5.3±1.6 | 2.2±0.5 | 1.6±0.1 | * |
| 130 | 3.6±0.6 | 1.8±0.2 | 0.8(N=1) | * |

| | | | | |
|---|---|---|---|---|
| * Not Determined - clotting occurred too fast for time measurement. ("N" refers to the mean of the 4 measurements made.) | | | | |

As Table 1 shows, clotting times at the lower thrombin concentration (e.g., 100 NIH U/ml) and low protein content (e.g., 50 mg/ml), were long. The mixture was also observed to be "runny". Higher concentrations of thrombin (e.g., 1000 NIH U/ml) generally clotted within the delivery device and therefore were considered unsuitable. Addition of CaCl₂ improved the appearance of the clot and generally shortened the clotting time.

Table 2 shows the effect of [CA⁺⁺] in the range of 0-60 mM. CaCl₂ solution was used to reconstitute the thrombin so the final [Ca⁺⁺], in the 1:1 mixture of FS is half that reported in the Table.

**TABLE 2**

| **EFFECT OF [Ca**^{**++**}**] ON TIME TO CLOT** | | | |
|---|---|---|---|
| | **Thrombin in NIH U/ml** | | |
| **Total Protein mg/ml** | **100** | **250** | **500** |
| 1. [Protein] = 50 mg/ml | | | |
| | | | |
| 0 mM ([Ca⁺⁺]) | 4.3±0.7 | 2.6±0.2 | 2.0±0.6 |
| 10 mM ([Ca⁺⁺]) | 2.4±0.3 | 1.8±0.2 | * |
| 20 mM ([Ca⁺⁺]) | 2.3±0.2 | 1.2±0.5 | * |
| 40 mM ([Ca⁺⁺]) | 2.6±0.2 | * | * |
| 60 mM ([Ca⁺⁺]) | 3.0±0.5 | * | * |

| 2. [Protein] = 90 mg/ml | | | |
|---|---|---|---|
| | | | |
| 0 mM ([Ca⁺⁺]) | 4.1±1.1 | 2.2±0.2 | 1.8±0.3 |
| 10 mM ([Ca⁺⁺]) | 2.9±0.3 | 2.8±0.3 | * |
| 20 mM ([Ca⁺⁺]) | 2.0±0.4 | 2.6±0.1 | * |
| 40 mM ([Ca⁺⁺]) | 2.5±0.2 | 1.9±0.1 | * |
| 60 mM ([Ca⁺⁺]) | 3.2±0.1 | * | * |

| 3. (Protein] = 110 mg/ml | | | |
|---|---|---|---|
| | | | |
| 0 mM ([Ca⁺⁺]) | 4.8±1.8 | 2.4±0.5 | 1.7±0.3 |
| 10 mM ([Ca⁺⁺]) | 2.9±0.4 | 2.4±1.2 | * |
| 20 mM ([Ca⁺⁺]) | 2.6±0.2 | * | * |
| 40 mM ([Ca⁺⁺]) | 2.6±0.7 | * | * |

| 4. [Protein] = 130 mg/ml | | | |
|---|---|---|---|
| | | | |
| 0 mM ([Ca⁺⁺]) | 3.1±0.8 | 1.5±0.1 | * |

| | | | |
|---|---|---|---|
| * Not Determined - clotting occurred too fast for time measurement [Protein] refers to the concentration of total protein in mg/ml in the formulation tested. | | | |

Based on the above results, it was concluded that a protein range of 90-130 mg/ml and a thrombin concentration of 250-500 NIH U/ml are appropriate for further studies. It was also apparent that additional calcium ion was needed to enhance the clot. Consequently, Ca⁺⁺ concentration was included as a variable in later evaluations.

### B. Rate of Cross-Linking

These studies focused on determining the role of Ca ions and the effect of [Ca⁺⁺] on the extent of fibrin polymerization as well as the rate of cross linking with time due to fibrin polymerization.

The cross-linking reaction of fibrin was tested in a system under reduced conditions which utilized SDS/PAGE. The resolving gels at 7.5% and the stacking gels at 3.75% were cast as described by Schwartz, *et al. (Journal of Clinical Investigation,* 50:1506, 1971).

Thrombin (Armour Pharmaceutical) was reconstituted with or without CaCl₂ solution at the desired molarity, i.e., 0, 2, 6, 12, 20, 40, or 60 mM. Fibrinogen was reconstituted with water, quickly mixed with the thrombin in a 12 x 75 mm test tube and sampled at the appropriate time periods for the studies. The clots were rinsed with 0.15M NaCI then dissolved in three times the clot volume of 9M urea containing 3% SDS and 3% β-mercaptoethanol by boiling in a water bath at 95 ± 5°C. The dissolved clot solutions were then stored at 5°C until the gel electrophoresis was performed.

The effect of CaCl₂ concentration was tested with thrombin at 500 NIH U/ml and fibrinogen at 90 mg/ml and 130 mg/ml after 10 minutes clotting time. The effect of Ca⁺⁺ concentration on the rate of disappearance of the γ-band to form the γ-γ dimer is shown in FIGURES 2 and 3.

As illustrated in FIGURES 2 and 3, the presence of Ca⁺⁺ is necessary for complete fibrin polymerization. As calcium ion in the range of 20-60 mM gives comparable results, a midpoint concentration of 40 mM was chosen to ensure optimal polymerization. Also, there was no significant difference in fibrin polymerization between the two fibrinogen concentrations (90 mg/ml and 130 mg/ml) when measured at the time point studied (10 minutes).

Four combinations of thrombin at 250 NIH U/ml or 500 NIH U/ml in 40 mM CaCl₂ and fibrinogen at 90 mg/ml or 130 mg/ml were tested to study the effect of varying the concentrations of the components. Samples were taken for gel electrophoresis after 10 minutes of clotting time. Gel electrophoresis of the four combinations of thrombin (in 40 mM CaCl₂) and fibrinogen did not reflect any significant differences at the time point studied (10 minutes).

The time study was conducted and sampled over 24 hours. FIGURE 4 shows the fibrin polymerization reaction as it progresses through a 24 hour period. Formation of the γ-γ dimer occurs very rapidly in the presence of Ca⁺⁺ (within one minute) as is shown in FIGURE 4. The α polymer is not detectable by this system until 10 minutes incubation time. As the α polymer increases with increasing incubation time (up to 24 hours) the α monomer band shows a corresponding decrease in intensity. To summarize, the time study demonstrates that initial polymerization (γ-γ dimer) occurs almost instantaneously as the reactants are mixed, with the α polymers forming more slowly. From this study it can be concluded that the presence of Ca ion is necessary for polymerization and that the results are similar to those previously reported in the literature (T. Seelich, *J. Head & Neck Pathol.,* 3:65-69, 1982; M. Schwartz, *et al., J.Clinical Inv.,* 50:1506-1513, 1971).

### C. Tensile Strength

The tensile strength of the fibrin sealant was evaluated by applying strain to the clot until rupture of the bulk material was observed and measuring the force needed in a tensile stress-strain system. In addition, the change in rupture stress as a function of varying the components in the polymerization mixture used to produce the sealant was studied.

To study the tensile strength of the Fibrin Sealant, a mold was designed based on that described by Nowotny, *et al. (Biomaterials,* 2:55, 1981) with some modifications. The newly designed mold was fabricated of transparent plastic to facilitate visual inspection of clot formation. Clotting was allowed to proceed in disposable clot holders for ease of cleaning.

The clot holders were obtained by cutting plastic disposable transfer pipets (SAMCO, San Fernando Mfg. Co.). Two small pieces of moistened sponge were used to anchor the clotting mixture at both ends. Disposable clot holders with the sponges in place were inserted through the end holders and into the mold, (end holders were included in the mold). A tensometer instrument (T10, Monsanto) was used to measure and record the peak rupture stress of the clots. Adapters for the T10 grippers were fabricated to hold the end holders.

The clots were formed by injecting equal volumes of fibrinogen and thrombin (with or without CaCl₂) using a dual syringe administration device (Fenwal) and a 3 inch 22 gauge needle. All bubbles were removed prior to placing the syringes in their holder. The needle was inserted through one sponge "top", through the mold and into the other sponge "bottom". Parafilm (American Can Co.) placed under the entire mold prevented leakage of excess mixture. As the clotting mixture filled the mold, the needle was withdrawn.

Approximately 2 to 5 minutes before testing, the clot was removed from the mold and placed in the T10 grippers. At testing time, the clot was stretched at a rate of 100 mm/min. The gauge length was set (somewhat arbitrarily) at 6.0 cm and the cross sectional area of the clot was 0.049 cm². The T10 reported the stress values in Kg/cm².

**TABLE 3**

| **EFFECT OF CaCl**_{**2**} **ON TENSILE STRENGTH OF FIBRIN SEALANT** | | | |
|---|---|---|---|
| | | **Tensile Strength Kg/cm**^{**2**} | |
| **Fgn.* Conc. mg/ml** | **CaCl**_{**2**} **mMol** | **@ 250 NIH U/ml Thrombin (N)** | **@ 500 NIH U/ml Thrombin (N)** |
| 90 | 0 | 0.93±0.136 (9) | 1.23±0.165 (8) |
| 110 | 0 | 1.07±0.1 50 (10) | 1.29±0.237 (9) |
| 130 | 0 | 0.94±0.157 (14) | 1.36±0.177 (16) |
| | | | |
| 90 | 10 | 1.65±0.380 (12) | 1.92±0.350 (8) |
| 110 | 10 | 2.60±0.540 (8) | 2.80±0.610 (8) |
| 130 | 10 | 2.35±0.760 (11) | 3.24±1.170 (8) |
| | | | |
| 90 | 20 | 1.65±0.406 (8) | 2.15±0.540 (8) |
| 110 | 20 | 2.26±0.530 (8) | 4.11±1.080 (8) |
| 130 | 20 | 2.23±0.489 (8) | 3.54±1.030 (8) |
| | | | |
| 90 | 40 | 1.74±0.410 (8) | 2.36±0.390 (11) |
| 110 | 40 | 2.36±0.660 (8) | 3.79±0.626 (8) |
| 130 | 40 | 2.63±0.670 (12) | 4.05±0.940 (9) |
| | | | |
| 90 | 60 | 2.10±0.38 (8) | 2.29±0.450 (8) |
| 110 | 60 | 3.00±0.690 (8) | 4.13±0.896 (8) |
| 130 | 60 | 3.69±0.787 (10) | 3.57±1.160 (9) |

| | | | |
|---|---|---|---|
| * Fibrinogen Lot #2830R129 | | | |

Measurements of tensile strength (peak stress) were taken 10 minutes after injection of the clotting mixture. The results in Table 3 show the effect of varying the CaCl₂ concentration when thrombin is 250 NIH U/ml or 500 NIH U/ml. Three concentrations of fibrinogen (90, 110, and 130 mg/ml) were tested. Each measurement of peak stress was the average of N determinations. A minimum of 8 readings were taken per point.

As shown in Table 3, in the absence of Ca⁺⁺, the clots had the lowest peak stress values at both thrombin concentrations and at all fibrinogen levels. Addition of Ca ions at 10-60 mM increased the tensile strength for all thrombin/fibrinogen concentrations. Generally, higher values were observed at the higher thrombin concentration, i.e., 500 NIH U/ml, of thrombin, which also gave somewhat similar values for [Ca⁺⁺] in the range of 20-60 mM (of Ca⁺⁺). The lowest standard deviation (SD) values were observed at 40 mM CaCl₂.

A second lot of human fibrinogen was tested to confirm these findings and the data were compared at 40 and 60 mM CaCl₂ and 500 NIH U/ml thrombin. The results of testing a second lot of fibrinogen showed generally similar values of peak stress particularly at the higher fibrinogen concentrations of 110 and 130 mg/ml and also showed higher values at 90 mg/ml.

Time studies of the clot tensile strength were performed over a 24 hour time period using fibrinogen at 90 mg/ml and 130 mg/ml with thrombin concentration at 500 NIH U/ml and CaCl₂ at 40 mM. Over a 24 hour period, the tensile strength showed no significant decrease in value. A gradual increase in peak stress was expected to occur as cross linking continued with time.

### D. Clot Lysis Studies

The length of time that a fibrin clot will remain solid when incubated at 37°C under sterile, moist conditions, with and without a plasminogen activator, was determined. Also tested was the effect on the clot longevity of adding protease inhibitor (Aprotinin) to the reaction mixture.

Sterile human fibrinogen solution was prepared using one of the following diluents:
a. sterile water (i.e., zero KIU/ml Aprotinin)
b. Aprotinin solution at 1000 KIU/ml
c. Aprotinin solution at 3000 KIU/ml
to yield one of three concentrations; 90, 110, or 130 mg/ml of total protein.

Thrombin was prepared by reconstituting with a 40 mM CaCl₂ solution to produce either a 250 or 500 NIH U/ml. Thus, six combinations of thrombin and fibrinogen were tested. Urokinase (Abbott) was prepared at 5 U/ml in normal saline.

Fibrin clots were formed by mixing equal volumes of fibrinogen (in H₂O or Aprotinin) and thrombin (in CaCl₂ solution) in cylindrical silicone tubing (5 mm inner diameter). The mixture was delivered using a dual syringe administration device (Fenwal). All the delivery devices and the silicone tubing were sterilized by autoclaving.

A 10 cm length of silicone tubing was sealed at one end using parafilm. Holding the tubing about 10° from vertical, the Fenwal device was used to inject the fibrinogen and thrombin rapidly into the tubing with the (22 g) needle tip barely penetrating the parafilm. After the clot had solidified for 20 minutes, the 10 cm silicone tubing was cut into 3 cm lengths to yield a clot volume of 590 µl. Each 3 cm segment was cut in half and the two halves were placed in one well of a sterile 24-well plate (Corning). Any segment that was found to contain air bubbles was discarded. The clot was extruded from the tubing by gently squeezing the tube at one end. It was rinsed with 1 ml of sterile saline then 1 ml of either urokinase or saline was added to the well and the plate was placed in a sterile, moist, 37°C incubator. Every 24 hours, the supernatants from each well were removed for testing using the Fibrin(ogen) Degradation Products agglutination kit (Baxter Dade).

The urokinase and saline solutions were replaced daily with fresh reagents before returning the plate to the 37°C incubator. All preparations and sampling of supernatants were performed under sterile conditions. The clots were visually inspected and their appearance noted. After 14 days the experiment was terminated. Total number of conditions tested was 36 and all conditions were performed in duplicate.

**TABLE 4**

| **CLOT LYSIS TIMES [+ UROKINASE. NO APROTININ]** | |
|---|---|
| **CONDITION** | **TIME CLOT LOST CYLINDRICAL SHAPE** |
| 130 mg/ml fibrinogen + 500 U/ml thrombin | Day 10 |
| 130 mg/ml fibrinogen + 250 U/ml thrombin | Day 8 |
| 110 mg/ml fibrinogen + 500 U/ml thrombin | Day 7 |
| 110 mg/ml fibrinogen + 250 U/ml thrombin | Day 11 |
| 90 mg/ml fibrinogen + 500 U/ml thrombin | Day 8 |
| 90 mg/ml fibrinogen + 250 U/ml thrombin | Day 8 |

Table 4 summarizes the clot lysis time (defined as the time clots lost their cylindrical shape) in the presence of urokinase when no Aprotinin was included. The observed range was 7-11 days with a mean of 8.66 ± 1.5d. Measurements of Fibrin(ogen) Degradation Products (FDP) showed a peak in activity that generally corresponded to or soon followed the time when the clots lost their well-defined shape.

When urokinase was deleted and the clots were incubated in normal saline only, in presence or absence of Aprotinin, the clots maintained their integrity during the entire observation period (i.e., 14 days). FDP measurements confirmed the absence of significant clot lysis.

This study shows that when the clot is not influenced by any plasminogen activators *in situ,* it should be expected to last for at least 14 days. When urokinase is present, the clots last a minimum of 7 days. These time periods may be sufficient for the healing mechanism to play its natural role. Thus, based on these results, it can be concluded that the trace levels of plasminogen in the fibrinogen preparations do not adversely affect the clot longevity and that the addition of a protease inhibitor, such as Aprotinin, is not necessary.

### EXAMPLE 3

### TFC in vivo TESTING

Studies were done to evaluate the optimal concentration of TFC using an *in vivo* model. Swiss Webster mice (20-25 g) were arranged in 10 groups of five for testing. In the protocol which was utilized, each animal was anesthetized, weighed, and a small piece of skin was removed from the back of the animal. The skin specimen was dipped in a saline solution and attached to a Gottlob device. Equal volumes of TFC and thrombin at various concentrations (Table 2) were then added simultaneously to the wound, the skin replaced onto the animal, and held in place for approximately two minutes.

The anesthetized animal was placed face down on a platform which was then positioned on a tensometer (Monsanto Company) and the Gottlob device attached to the grippers. The tensometer parameters were set to: (1) area: 1.76 cm²; (2) speed: 10.0 mm/min.; (3) gauge: 1.0 cm; (4) stress range: 500.0%. The force required to separate the piston (with the skin specimen) from the back of the animal was recorded in g/cm². The data from these experiments were statistically evaluated using RS1/Discover software (BBN Software Corp., Cambridge, MA). Analysis of the results of this study indicated that TFC at 120-130 mg/mL and thrombin at 250 U/mL gave maximal adhesion responses.

The ability of the clot to adhere to tissue *in vivo* is important in maintaining hemostasis. In this experiment, a maximum adhesion response occurred within the range of reagents tested, confirming the *in vitro* findings of Example 2.

The preceding examples and description are provided to assist in understanding the present invention and, as such, are intended to be exemplary only, not limiting.

## Claims

1. A method for preparing a hemostasis promoting composition derived from human plasma or plasma fractions wherein the human plasma or plasma fractions comprise fibrinogen, Factor VIII, and plasminogen, comprising:
(a) providing a cryoprecipitated plasma preparation from the human plasma or plasma fractions;
(b) separating the cryoprecipitate from the cryoprecipitated plasma preparation;
(c) forming a cold-precipitate by dissolving the cryoprecipitate in a medium and cooling the medium, the cold-precipitate having significantly less Factor VIII than the cryoprecipitate;
(d) suspending the cold-precipitate of step (c) in a salt-containing buffer to eliminate prothrombin complex from the cryoprecipitate;
(e) treating the supernatant obtained from the suspension in step (d) by affinity-chromatography on a lysine-bound solid matrix to allow plasminogen to adsorb thereon;
(f) collecting the fraction containing no higher than trace levels of plasminogen;
(g) contacting the fraction of step (f) with a virally inactivating effective amount of an antiviral agent;
(h) removing the antiviral agent from the virally inactivated material obtained in step (g); and
(i) recovering a fibrinogen-containing composition.

2. The method of claim 1, wherein the cryoprecipitate in step (c) is initially dissolved in water having a temperature from about 20°C to about 35°C and the cooling is maintained at a temperature of 10°C ± 5°C.

3. The method of claim 1, wherein calcium ion is added to the dissolved cryoprecipitate in step (c) at a concentration sufficient to enhance the precipitation of fibrinogen.

4. The method of claim 3, wherein the calcium ion is in the form of a calcium salt.

5. The method of claim 4, wherein the calcium salt is CaCl₂.

6. The method of claim 5, wherein the CaCl₂ is added to a final concentration from 0.001 mM to 1 mM.

7. The method of claim 6, wherein the CaCl₂ is added to a final concentration of 0.040 mM.

8. The method of claim 1, further including the step of (c') prior to step (d), wherein the step (c') comprises treating the cold-precipitate with a protease inhibitor in a concentration sufficient to inhibit thrombin activity.

9. The method of claim 8, wherein the protease inhibitor is selected from the group consisting of PPACK, heparin cofactor II, hirudin, and anti-thrombin III (AT III).

10. The method of claim 9, wherein the concentration of PPACK is from 0.75 µM to 1.75µM.

11. The method of claim 1, wherein the solid matrix of step (c) is agarose.

12. The method of claim 1, further including the step of (f') prior to step (g), wherein the step (f') comprises treating the fractions by addition of polyethylene glycol (PEG).

13. The method of claim 12, wherein the PEG has a molecular weight from about 3,000 Daltons to about 8,000 Daltons.

14. The method of claim 13, wherein the PEG has a molecular weight of about 3350 Daltons.

15. The method of claim 12, wherein in step (f') the PEG is added to a final concentration from 3% to 7%.

16. The method of claim 12, wherein in step (f') the PEG is added to a final concentration of 4% (w/w).

17. The method of claim 1, wherein in step (g) the anti-viral agent is a detergent.

18. The method of claim 17, wherein the detergent is non-ionic.

19. The method of claim 18, wherein the concentration of the detergent is from 0.001% (v/v) to 10% (v/v).

20. The method of claim 19, wherein the concentration of detergent is from 0.01% (v/v) to 2% (v/v).

21. The method of claim 17, wherein a mixture of detergents is utilized.

22. The method of claim 21, wherein the mixture is non-ionic.

23. The method of claim 22, wherein the mixture includes a sulfated oxyethylated alkylphenol and a polyoxyethylated ester derivative.

24. The method of claim 23, wherein the sulfated oxyethylated alkylphenol is Triton X-100.

25. The method of claim 23, wherein the polyoxyethylated ester derivative is Polysorbate-80.

26. The method of claim 22, wherein the total final concentration of detergent mixture is from 1% (v/v) to 2% (v/v).

27. The method of claim 22, wherein the total final concentration of detergent mixture is from 1% (v/v) to 1.5% (v/v).

28. The method of claim 22, wherein the total final concentration of detergent mixture is from 1.2% (v/v) to 1.4% (v/v).

29. The method of claim 24, wherein the final concentration of Triton x-100 is from 0.8% (v/v) to 1.2% (v/v).

30. The method of claim 25, wherein the final concentration of Polysorbate-80 is from 0.2% (v/v) to 0.4% (v/v).

31. The method of claim 1, wherein in step (g) the anti-viral agent is an organic solvent.

32. The method of claim 31, wherein the organic solvent is an alkyl phosphate.

33. The method of claim 32, wherein the alkyl posphate is tri (n-butyl) phosphate.

34. The method of claim 33, wherein the final concentration of tri (n-butyl) phosphate is from 0.2% (v/v) to 0.4% (v/v).

35. The method of claim 17 or claim 31, wherein the anti-viral agent is removed by adsorbing fibrinogen on an anionic exchange resin and by washing the anti-viral agent not bound to the resin.

36. The method of claim 35, wherein the moiety in the anionic exchange resin is diethylaminoethyl (DEAE).

37. The method of claim 36, wherein the DEAE is attached to cellulose.

38. The method of claim 37, wherein the anion exchange matrix is DE 52 cellulose.

39. The method of claim 35, wherein the material bound to the anion exchange resin is eluted with 0.25 M to 0.40 M NaCl.

40. The method of claim 1, which further comprises the steps of:
(j) adding a stabilizer and/or a solubilizer to the recovered composition of step (i);
(k) concentrating the composition of step (j) to about 20% to about 50% of its original volume;
(I) diluting the concentrate of step (k) to its preconcentration volume;
(m) concentrating the composition of step (I) to about 3 g% to about 5 g% (w/v), and
(n) sterile processing the composition.

41. The method of claim 40, wherein in step (n) the composition is additionally lyophilized.

42. The method of claim 40, wherein the pre-lyophilization volume of the composition is about 3 times the volume of the composition after the lyophilizate is resuspended.

43. The method of claim 42, wherein NaCI is present in the resuspended lyophilizate in a concentration of 0.1 M to 0.2 M.

44. The method of claim 40, wherein in step (j) the stabilizer is human serum albumin (HSA).

45. The method of claim 44, wherein the HSA is from 70mg to 100mg per gram of protein in the eluate.

46. The method of claim 40, wherein in step (j) the solubilizer is a polyoxyethylated ester derivative.

47. The method of claim 46, wherein the polyoxyethylated ester derivative is Polysorbate-80.

48. The method of claim 47, wherein the Polysorbate-80 is from about 10mg to about 20mg per gram of protein in the eluate.

49. The method of claim 40, wherein in step (k) and in step (m), the concentration is accomplished by ultrafiltration.

50. The method of claim 49, wherein the ultrafiltration utilizes a membrane with a 30,000 Dalton molecular weight cut-off.

51. A hemostasis promoting composition derived from human plasma or plasma fractions wherein the composition:
(a) has therein from 75% to 95% human fibrinogen;
(b) has therein from 10 to 40 units/ml human F XIII;
(c) is virally inactive;
(d) is substantially non-pyrogenic;
(e) has therein no higher than trace levels of plasminogen;
(f) is free of protein which is encoded by a non-human DNA sequence;
(g) is essentially free of thrombin; and
(h) does not contain a fibrinolysis inhibitor.

52. The composition of Claim 51, wherein the composition is lyophilized.

53. The composition of Claim 51 which further comprises a stabilizer.

54. The composition of Claim 53 wherein the stabilizer is HSA.

55. The composition of Claim 53 wherein the HSA is at a concentration from 0.5 g% to 1.5 g%.

56. The composition of Claim 51 which further comprises a solubilizer.

57. The composition of Claim 56 wherein the solubilizer is a polyoxyethylated ester derivative.

58. The composition of Claim 57 wherein the polyoxyethlayted ester derivative is Polysorbate-80.

59. The composition of Claim 58 wherein the Polysorbate-80 is present in an amount of from 0.1% to 0.3%.

60. The composition of Claim 51 which further comprises a therapeutically effective amount of a drug.

61. The composition of Claim 51 which further comprises a diagnostically effective amount of a diagnostic agent.

62. Use of a composition as claimed in any one of Claims 51 to 61 in the manufacture of a fibrin sealant.

63. A fibrin sealant comprising:
(i) a hemostasis promoting composition derived from human plasma or plasma fractions wherein the composition:
(a) has therein from 75% to 95% human fibrinogen;
(b) has therein from 10 to 40 units/ml human F XIII;
(c) is virally inactive;
(d) is essentially non-pyrogenic;
(e) has therein no higher than trace levels of plasminogen;
(f) is free of protein which is encoded by a non-human DNA sequence;
(g) is essentially free of thrombin; and
(h) does not contain a fibrinolysis inhibitor.
(ii) thrombin; and
(iii) calcium.

## Patentansprüche

1. Verfahren zur Herstellung einer Hämostase-fördernden Zusammensetzung, die von menschlichem Plasma oder Plasmafraktionen abgeleitet ist, wobei das menschliche Plasma oder die Plasmafraktionen Fibrinogen, Faktor VIII und Plasminogen umfassen, umfassend die Stufen:
(a) Bereitstellen einer kryopräzipitierten Plasma-Zubereitung aus dem menschlichen Plasma oder den Plasmafraktionen;
(b) Abtrennen des Kryopräzipitats von der kryopräzipitierten Plasma-Zubereitung;
(c) Ausbilden eines Kältepräzipitats durch Auflösen des Kryopräzipitats in einem Medium und Abkühlen des Mediums, wobei das Kältepräzipitat wesentlich weniger Faktor VIII aufweist als das Kryopräzipitat;
(d) Suspendieren des Kältepräzipitats der Stufe (c) in einem Salz-enthaltenden Puffer, um Prothrombinkomplexe aus dem Kryopräzitat zu entfernen;
(e) Behandeln des aus der Suspension in Stufe (d) erhaltenen Überstandes mittels Affinitätschromatographie an einer Lysin-gebundenen festen Matrix, um daran Plasminogen adsorbieren zu lassen;
(f) Sammeln der nicht mehr als Spuren Plasminogen enthaltenden Fraktion;
(g) In-Kontakt-bringen der Fraktion von Stufe (f) mit einer virusinaktivierend-wirksamen Menge eines antiviralen Mittels;
(h) Entfernen des antiviralen Mittels aus dem in Stufe (g) erhaltenen virusinaktivierten Material; und
(i) Gewinnen der Fibrinogen-enthaltenden Zusammensetzung.

2. Verfahren nach Anspruch 1,
wobei das Kryopräzipitat in Stufe (c) zuerst in Wasser mit einer Temperatur von ca. 20 °C bis ca. 35 °C gelöst wird und das Abkühlen bei einer Temperatur von 10 °C bis ± 5 °C durchgeführt wird.

3. Verfahren nach Anspruch 1,
wobei zu dem gelösten Kryopräzipitat in Stufe (c) Calciumionen in einer Konzentration zugegeben werden, die ausreicht, um die Ausfällung von Fibrinogen zu erhöhen.

4. Verfahren nach Anspruch 3,
wobei die Calciumionen in Form eines Calciumsalzes vorliegen.

5. Verfahren nach Anspruch 4,
wobei das Calciumsalz CaCl₂ ist.

6. Verfahren nach Anspruch 5,
wobei CaCl₂ bis zu einer Endkonzentration von 0,001 mM bis 1 mM zugegeben wird.

7. Verfahren nach Anspruch 6,
wobei das CaCl₂ bis zu einer Endkonzentration von 0,040 mM zugefügt wird.

8. Verfahren nach Anspruch 1,
das außerdem die Stufe (c') vor der Stufe (d) umfaßt, wobei die Stufe (c') die Behandlung des Kältepräzipitats mit einem Proteaseinhibitor bei einer Konzentration umfaßt, die ausreicht, um die Thrombinaktivität zu inhibieren.

9. Verfahren nach Anspruch 8,
wobei der Proteaseinhibitor ausgewählt ist aus der Gruppe, die aus PPACK, Heparincofaktor II, Hirudin und Antithrombin III (AT III) besteht.

10. Verfahren nach Anspruch 9,
wobei die Konzentration an PPACK 0,75 µM bis 1,75 µM beträgt.

11. Verfahren nach Anspruch 1,
wobei die feste Matrix der Stufe (c) Agarose ist.

12. Verfahren nach Anspruch 1,
das außerdem die Stufe (f') vor der Stufe (g) umfaßt, wobei die Stufe (f') die Behandlung der Fraktionen durch Zugabe von Polyethylenglykol (PEG) umfaßt.

13. Verfahren nach Anspruch 12,
wobei das PEG ein Molekulargewicht von ca. 3000 Dalton bis ca. 8000 Dalton aufweist.

14. Verfahren nach Anspruch 13,
wobei das PEG ein Molekulargewicht von ca. 3350 Dalton aufweist.

15. Verfahren nach Anspruch 12,
wobei in Stufe (f') das PEG bis zu einer Endkonzentration von 3 % bis 7 % zugegeben wird.

16. Verfahren nach Anspruch 12,
wobei in Stufe (f') das PEG bis zu einer Endkonzentration von 4 % (Gew/Gew) zugegeben wird.

17. Verfahren nach Anspruch 1,
wobei in Stufe (g) das antivirale Mittel ein Detergens ist.

18. Verfahren nach Anspruch 17,
wobei das Detergens nicht-ionisch ist.

19. Verfahren nach Anspruch 18,
wobei die Konzentration des Detergens 0,001 % (V/V) bis 10 % (V/V) beträgt.

20. Verfahren nach Anspruch 19,
wobei die Konzentration des Detergens 0,01 % (V/V) bis 2 % (V/V) beträgt.

21. Verfahren nach Anspruch 17,
wobei eine Mischung aus Detergentien verwendet wird.

22. Verfahren nach Anspruch 21,
wobei die Mischung nicht-ionisch ist.

23. Verfahren nach Anspruch 22,
wobei die Mischung ein sulfatiertes oxyethyliertes Alkylphenol und ein polyoxyethyliertetes Esterderivat umfaßt.

24. Verfahren nach Anspruch 23,
wobei das sulfatierte oxyethylierte Alkylphenol Triton X-100 ist.

25. Verfahren nach Anspruch 23,
wobei das polyoxyethylierte Esterderivat Polysorbat 80 ist.

26. Verfahren nach Anspruch 22,
wobei die gesamte Endkonzentration der Detergentienmischung 1 % (V/V) bis 2 % (V/V) beträgt.

27. Verfahren nach Anspruch 22,
wobei die gesamte Endkonzentration der Detergentienmischung 1 % (V/V) bis 1,5 % (V/V) beträgt.

28. Verfahren nach Anspruch 22,
wobei die gesamte Endkonzentration der Detergentienmischung 1,2 % (V/V) bis 1,4 % (V/V) beträgt.

29. Verfahren nach Anspruch 24,
wobei die Endkonzentration an Triton X-100 einen Wert von 0,8 % (V/V) bis 1,2 % (V/V) hat.

30. Verfahren nach Anspruch 25,
wobei die Endkonzentration an Polysorbat 80 einen Wert von 0,2 % (V/V) bis 0,4 % (V/V) hat.

31. Verfahren nach Anspruch 1,
wobei in Stufe (g) das antivirale Mittel ein organisches Lösungsmittel ist.

32. Verfahren nach Anspruch 31,
wobei das organische Lösungsmittel ein Alkylphosphat ist.

33. Verfahren nach Anspruch 32,
wobei das Alkylphosphat Tri-(n-butyl)-phosphat ist.

34. Verfahren nach Anspruch 33,
wobei die Endkonzentration an Tri-(n-butyl)-phosphat 0,2 % (V/V) bis 0,4 % (V/V) beträgt.

35. Verfahren nach Anspruch 17 oder Anspruch 31,
wobei das antivirale Mittel durch Adsorbieren von Fibrinogen an einem anionischen Austauscherharz und Wegwaschen des nicht an das Harz gebundenen antiviralen Mittels entfernt wird.

36. Verfahren nach Anspruch 35,
wobei der Rest im anionischen Austauscherharz Diethylaminoethyl (DEAE) ist.

37. Verfahren nach Anspruch 36,
wobei das DEAE an Cellulose gebunden ist.

38. Verfahren nach Anspruch 37,
wobei die Anionenaustauscher-Matrix DE 52-Cellulose ist.

39. Verfahren nach Anspruch 35,
wobei das an das Anionenaustauscherharz gebundene Material mit 0,25 M bis 0,40 M NaCl eluiert wird.

40. Verfahren nach Anspruch 1,
das außerdem die folgenden Stufen umfaßt:
(j) Zugeben eines Stabilisators und/oder eines Solubilisators zu der in Stufe (i) erhaltenen Zusammensetzung;
(k) Aufkonzentrieren der Zusammensetzung der Stufe (j) auf ca. 20 % bis ca. 50 % ihres ursprünglichen Volumens;
(1) Verdünnen des Konzentrats der Stufe (k) auf sein Volumen vor der Aufkonzentrierung;
(m) Aufkonzentrieren der Zusammensetzung der Stufe (1) auf ca. 3 g% bis ca. 5 g% (Gew/Vol), und
(n) sterile Aufarbeitung der Zusammensetzung.

41. Verfahren nach Anspruch 40,
wobei in Stufe (n) die Zusammensetzung zusätzlich lyophilisiert wird.

42. Verfahren nach Anspruch 40,
wobei das Volumen der Zusammensetzung vor der Lyophilisierung etwa das dreifache des Volumens der Zusammensetzung nach dem Resuspendieren des Lyophilisats beträgt.

43. Verfahren nach Anspruch 42,
wobei in dem resuspendierten Lyophilisat NaCl in einer Konzentration von 0,1 M bis 0,2 M vorhanden ist.

44. Verfahren nach Anspruch 40,
wobei in Stufe (j) der Stabilisator menschliches Serumalbumin (HSA) ist.

45. Verfahren nach Anspruch 44,
wobei das HSA in einer Menge von 70 mg bis 100 mg pro Gramm Protein im Eluat enthalten ist.

46. Verfahren nach Anspruch 40,
wobei in Stufe (j) der Solubilisator ein polyoxyethyliertes Esterderivat ist.

47. Verfahren nach Anspruch 46,
wobei das polyoxyethylierte Esterderivat Polysorbat 80 ist.

48. Verfahren nach Anspruch 47,
wobei das Polysorbat 80 in einer Menge von ca. 10 mg bis ca. 20 mg pro Gramm Protein im Eluat enthalten ist.

49. Verfahren nach Anspruch 40,
wobei in Stufe (k) und in Stufe (m) das Aufkonzentrieren durch Ultrafiltration erreicht wird.

50. Verfahren nach Anspruch 49,
wobei die Ultrafiltration eine Membran mit einer 30000 Dalton-Molekulargewichts-Ausschlußgrenze verwendet.

51. Hämostase-fördernde Zusammensetzung, die von menschlichem Plasma oder Plasmafraktionen abgeleitet ist, wobei die Zusammensetzung:
(a) darin 75 % bis 95 % menschliches Fibrinogen enthält;
(b) darin 10 bis 40 Einheiten/ml von menschlichem F XIII enthält;
(c) virusinaktiv ist;
(d) im wesentlichen nicht-pyrogen ist;
(e) darin nicht mehr als Spuren von Plasminogen enthält;
(f) frei ist von Protein, das durch eine nicht-menschliche DNA-Sequenz kodiert wird;
(g) im wesentlichen frei ist von Thrombin; und
(h) keinen Fibrinolyse-Inhibitor enthält.

52. Zusammensetzung nach Anspruch 51,
wobei die Zusammensetzung lyophilisiert ist.

53. Zusammensetzung nach Anspruch 51,
wobei sie ferner einen Stabilisator aufweist.

54. Zusammensetzung nach Anspruch 53,
wobei der Stabilisator HSA ist.

55. Zusammensetzung nach Anspruch 53,
wobei das HSA in einer Konzentration von 0,5 g% bis 1,5 g% vorliegt.

56. Zusammensetzung nach Anspruch 51,
wobei sie ferner einen Solubilisator umfaßt.

57. Zusammensetzung nach Anspruch 56,
wobei der Solubilisator ein polyoxyethyliertes Esterderivat ist.

58. Zusammensetzung nach Anspruch 57,
wobei das polyoxyethylierte Esterderivat Polysorbat 80 ist.

59. Zusammensetzung nach Anspruch 58,
wobei das Polysorbat 80 in einer Menge von 0,1 % bis 0,3 % vorliegt.

60. Zusammensetzung nach Anspruch 51,
die außerdem eine therapeutisch wirksame Menge eines Arzneimittels aufweist.

61. Zusammensetzung nach Anspruch 51,
die außerdem eine diagnostisch wirksame Menge eines Diagnostikums umfaßt.

62. Verwendung einer Zusammensetzung nach einem der Ansprüche 51 bis 61 zur Herstellung eines Fibrinklebers.

63. Fibrinkleber, der folgendes aufweist:
(i) eine Hämostase-fördernde Zusammensetzung, die von menschlichem Plasma oder Plasmafraktionen abgeleitet ist, wobei die Zusammensetzung:
(a) darin 75 % bis 95 % menschliches Fibrinogen enthält;
(b) darin 10 bis 40 Einheiten/ml von menschlichem F XIII enthält;
(c) virusinaktiv ist;
(d) im wesentlichen nicht-pyrogen ist;
(e) darin nicht mehr als Spuren von Plasminogen enthält;
(f) frei ist von Protein, das durch eine nichtmenschliche DNA-Sequenz kodiert wird;
(g) im wesentlichen frei ist von Thrombin; und
(h) keinen Fibrinolyse-Inhibitor enthält;
(ii) Thrombin; und
(iii) Calcium.

## Revendications

1. Procédé de préparation d'une composition favorisant l'hémostase dérivée de plasma ou de fractions plasmatiques humain(es) où le plasma humain ou les fractions plasmatiques humain(es) comprend(ennent) du fibrinogène, du facteur VIII et un plasminogène, dans lequel :
(a) on fournit une préparation de plasma cryoprécipitée provenant du plasma ou des fractions plasmatiques humain(es) ;
(b) on sépare le cryoprécipité de la préparation de plasma cryoprécipité ;
(c) on forme un précipité froid en dissolvant le cryoprécipité dans un milieu et en refroidissant le milieu, le précipité froid comportant significativement moins de facteur VIII que le cryoprécipité ;
(d) on met en suspension le précipité froid de l'étape (c) dans un tampon contenant un sel pour éliminer le complexe de prothrombine du cryoprécipité ;
(e) on traite le surnageant obtenu à partir de la suspension dans l'étape (d) par une chromatographie d'affinité sur une matrice de solide liée à la lysine pour permettre au plasminogène de s'y adsorber ;
(f) on recueille la fraction de plasminogène ne contenant pas plus que des traces de sel ;
(g) on met en contact la fraction de l'étape (f) avec une quantité efficace viralement inactivante d'un agent antiviral ;
(h) on retire l'agent antiviral du matériau viralement inactivé obtenu dans l'étape (g) ; et
(i) on recueille une composition contenant du fibrinogène.

2. Procédé de la revendication 1, dans lequel on dissout initialement le cryoprécipité de l'étape (c) dans de l'eau ayant une température d'environ 20°C à environ 35°C et on maintient le refroidissement à une température de 10°C ± 5°C.

3. Procédé de la revendication 1, dans lequel on ajoute de l'ion calcium au cryoprécipité dissous dans l'étape (c) à une concentration suffisante pour accroître la précipitation du fibrinogène.

4. Procédé de la revendication 3, dans lequel l'ion calcium est sous la forme d'un sel de calcium.

5. Procédé de la revendication 4, dans lequel le sel de calcium est CaCl₂.

6. Procédé de la revendication 5, dans lequel on ajoute CaCl₂ à une concentration finale de 0,001 mM à 1 mM.

7. Procédé de la revendication 6, dans lequel on ajoute le CaCl₂ à une concentration finale de 0,040 mM.

8. Procédé de la revendication 1, incluant en outre l'étape de (c') avant l'étape (d), où l'étape (c') comprend le traitement du précipité froid avec un inhibiteur de protéase à une concentration suffisante pour inhiber l'activité de thrombine.

9. Procédé de la revendication 8, dans lequel l'inhibiteur de protéase est choisi dans le groupe constitué par le PPACK, le cofacteur d'héparine II, l'hirudine et l'antithrombine III (AT III).

10. Procédé de la revendication 9, dans lequel la concentration de PPAC est de 0,75 µm à 1,75 µm.

11. Procédé de la revendication 1, dans lequel la matrice solide de l'étape (c) est l'agarose.

12. Procédé de la revendication 1, incluant en outre l'étape (f') avant l'étape (g), où l'étape (f') comprend le traitement des fractions par addition de polyéthylène glycol (PEG).

13. Procédé selon la revendication 12, dans lequel le PEG a un poids moléculaire compris entre environ 3000 daltons et environ 8000 daltons.

14. Procédé de la revendication 13, dans lequel le PEG a un poids moléculaire d'environ 3550 daltons.

15. Procédé de la revendication 12, dans lequel dans l'étape (f') le PEG est ajouté à une concentration finale de 3 % à 7 %.

16. Procédé de la revendication 12, dans lequel dans l'étape (f') le PEG est ajouté à une concentration finale de 4 % (p/p).

17. Procédé selon la revendication 1, dans lequel dans l'étape (g) l'agent antiviral est un détergent.

18. Procédé selon la revendication 17, dans lequel le détergent est non ionique.

19. Procédé de la revendication 18, dans lequel la concentration du détergent est de 0,001 % v/v à 10 % v/v.

20. Procédé de la revendication 19, dans lequel la concentration de détergent est de 0,01 % v/v à 2 % v/v.

21. Procédé de la revendication 17 dans lequel on utilise un mélange de détergents.

22. Procédé de la revendication 21 dans lequel le mélange est non ionique.

23. Procédé de la revendication 22, dans lequel le mélange inclut un alkylphénol oxyéthylé sulfaté et un dérivé ester polyoxyéthylé.

24. Procédé de la revendication 23, dans lequel l'alkylphénol oxyéthylé sulfaté est le Triton X-100.

25. Procédé de la revendication 23 dans lequel le dérivé ester polyoxyéthylé est le polysorbate 80.

26. Procédé de la revendication 22 dans lequel la concentration finale totale de mélanges de détergents est de 1 % v/v à 2 % v/v.

27. Procédé de la revendication 22 dans lequel la concentration finale totale des mélanges détergents est de 1 % v/v à 1,5 % v/v.

28. Procédé de la revendication 22, dans lequel la concentration finale totale des mélanges détergents est de 1,2 % v/v à 1,4 % v/v.

29. Procédé de la revendication 24, dans lequel la concentration finale de Triton-X 100 est de 0,8 % v/v à 1,2 % v/v.

30. Procédé de la revendication 29, dans lequel la concentration finale de polysorbate 80 est de 0,2 % v/v à 0,4 % v/v.

31. Procédé de la revendication 1 dans lequel dans l'étape (g) l'agent antiviral est un solvant organique.

32. Procédé de la revendication 31 dans lequel le solvant organique est un phosphate d'alkyle.

33. Procédé de la revendication 32 dans lequel le phosphate d'alkyle est le phosphate de tri(n-butyle).

34. Procédé de la revendication 33, dans lequel la concentration finale de phosphate de tri-n-butyle est de 0,2 % v/v à 0,4 % v/v.

35. Procédé de la revendication 17 ou de la revendication 31 dans lequel on retire l'agent antiviral en adsorbant les fibrinogènes sur une résine échangeuse d'anions et en lavant l'agent antiviral non lié à la résine.

36. Procédé de la revendication 35 dans lequel la partie qui se trouve dans la résine échangeuse anionique est le diaminoéthyle (DEAE).

37. Procédé selon la revendication 36, dans lequel le DEAE est attaché à de la cellulose.

38. Procédé selon la revendication 37 dans lequel la matrice échangeuse d'anions est la DE 52 cellulose.

39. Procédé de la revendication 35 dans lequel le matériau lié à la résine échangeuse d'anion est élué avec NaCl 0,25 M à 0,40 M.

40. Procédé de la revendication 1, qui comprend en outre les étapes de :
(j) addition d'un stabilisateur et/ou d'un agent solubilisant à la composition recueillie de l'étape (i)
(k) concentration de la composition de l'étape (j) à environ 20 % à environ 50 % de son volume d'origine ;
(1) dilution du concentré d'étape (k) à son volume antérieur à la concentration ;
(m) concentration de la composition de l'étape (1) à environ 3 g% en poids à environ 5 g% (p/v) et
(n) traitement stérile de la composition.

41. Procédé de la revendication 40, dans lequel dans l'étape (n) la composition est en outre lyophilisée.

42. Procédé de la revendication 40, dans lequel le volume de prélyophilisation de la composition est environ trois fois le volume de la composition après remise en suspension du produit de lyophilisation.

43. Procédé de la revendication 42 dans lequel NaCl est présent dans le lyophilisant mis en suspension à une concentration de 0,1 M à 0,2 M.

44. Procédé de la revendication 40 dans lequel dans l'étape (j) le stabilisateur est de la sérum albumine humaine (SAH).

45. Procédé de la revendication 44, dans lequel la SAH est de 70 mg à 100 mg/g de protéine dans l'éluat.

46. Procédé de la revendication 40 dans lequel dans l'étape (j) le stabilisateur est un dérivé ester polyoxyéthylé.

47. Procédé de la revendication 46 dans lequel le dérivé ester polyoxyéthylé est le polysorbate 80.

48. Procédé de la revendication 47 dans lequel le polysorbate 80 est à environ 10 mg à environ 20 mg/g de protéine dans l'éluat.

49. Procédé de la revendication 40, dans lequel dans l'étape (a) et dans l'étape (m) la concentration est obtenue par ultrafiltration.

50. Procédé de la revendication 49 dans lequel l'ultrafiltration utilise une membrane d'un seuil de poids moléculaire de 30 000 daltons.

51. Composition favorisant l'hémostase dérivée de plasma ou de fractions plasmatiques humain(es), où la composition :
(a) comporte 75 % à 95 % de fibrinogène humain ;
(b) comporte de 10 à 40 unités/ml de F XIII humain ;
(c) est viralement inactive ;
(d) est sensiblement non pyrogène ;
(e) ne comporte pas plus que des niveaux traces de plasminogène ;
(f) est dépourvue de protéine qui est encodée par une séquence d'ADN non humaine ;
(g) est essentiellement dépourvue de thrombine ; et
(h) ne contient pas d'inhibiteur de fibrinolyse.

52. Composition de la revendication 51, où la composition est lyophilisée.

53. Composition de la revendication 51 qui comprend en outre un stabilisateur.

54. Composition de la revendication 53 dans lequel le stabilisateur est la SAH.

55. Composition de la revendication 53 dans lequel la SAH est à une concentration de 0,5 g% à 1,5 g%.

56. Composition de la revendication 51 qui comprend en outre un agent solubilisant.

57. Composition de la revendication 56 dans laquelle l'agent solubilisant est un dérivé ester polyoxyéthylé.

58. Composition de la revendication 57 dans laquelle le dérivé ester polyoxyéthylé est le Polysorbate-80.

59. Composition de la revendication 58 dans laquelle le Polysorbate-80 est présent en une quantité de 0,1 % à 0,3 %.

60. Composition de la revendication 51 qui comprend en outre une quantité thérapeutiquement efficace d'un médicament.

61. Composition de la revendication 51 qui comprend en outre une quantité efficace pour le diagnostic d'un agent de diagnostic.

62. utilisation d'une composition selon l'une quelconque des revendications 51 à 61 pour la fabrication d'un agent obturant à base de fibrine.

63. Agent obturant à base de fibrine comprenant :
(i) une composition favorisant l'hémostase dérivée de plasma ou de fractions plasmatiques humain(es) où la composition :
(a) comporte de 75 % à 95 % de fibrinogène humain ;
(b) comporte de 10 à 40 unités/ml de F XIII humain ;
(c) est viralement inactive,
(d) est essentiellement non pyrogène ;
(e) ne comprend pas plus que des traces de plasminogène;
(f) est dépourvue de protéine qui est encodée par une séquence d'ADN non humaine ;
(g) est essentiellement dépourvue de thrombine et
(h) ne contient pas d'inhibiteur de fibrinolyse ;
(ii) de la thrombine ; et
(iii) du calcium.
